# FASCICULE DE BREVET EUROPEEN

(11) **EP 2 012 840 B1**
(45) Date de publication et mention de la délivrance du brevet: **08.08.2012**
(21) Numéro de dépôt: 07731962.2
(22) Date de dépôt: 27.04.2007
(51) Int. Cl.: A61L 2/26, B65B 55/02, A61J 1/00

(54) **SYSTEME DE CONDITIONNEMENT STERILISABLE PAR AUTOCLAVAGE ET SON PROCEDE DE FABRICATION**
DURCH AUTOKLAVIEREN STERILISIERBARES VERPACKUNGSSYSTEM UND HERSTELLUNGSVERFAHREN DAFÜR
PACKAGING SYSTEM THAT CAN BE STERILIZED BY AUTOCLAVING AND MANUFACTURING PROCESS THEREOF

(30) Priorité: 28.04.2006 FR 0651529
(43) Date de publication de la demande: 14.01.2009
(73) Titulaire: Bristol-myers Squibb, 92500 Rueil Malmaison (FR)
(72) Inventeur: GAUTIER, Régis, F-47240 Bon Encontre (FR); BERTOSSI, Serge, F-47550 Boe (FR)
(74) Mandataire: Hubert, Philippe
(86) Numéro de dépôt international: PCT/FR2007/051191
(87) Numéro de publication internationale: WO 2007/125261

(56) Documents cités:
- EP-A1- 0 067 420
- EP-A1- 0 109 983
- DE-A1- 4 342 329

## Description

La présente invention a pour objet un nouveau système de conditionnement stérilisable par autoclave d'un produit aqueux contenant au moins une substance susceptible de se dégrader, notamment par contact avec un gaz ou sous l'effet d'un rayonnement. Elle concerne également son procédé de fabrication.

L'invention trouve notamment application pour le conditionnement et la stérilisation dans un récipient souple, transparent et clos formé d'une matière plastique à base de poly(chlorure de vinyle) (PVC), de produits pharmaceutiques contenant au moins une substance susceptible de se dégrader par contact avec un gaz, notamment l'oxygène, et se présentant sous la forme de solutions, d'émulsions ou suspensions aqueuses.

Les matières plastiques à base de PVC, notamment utilisées dans le domaine pharmaceutique, présentent une forte perméabilité aux gaz à haute température.

De ce fait, l'oxygène de l'atmosphère est susceptible de s'introduire à l'intérieur du récipient à travers sa paroi, lors des opérations de stérilisation auxquelles les produits pharmaceutiques sont généralement soumis avant usage, et de provoquer ainsi la dégradation des constituants sensibles à l'oxygène éventuellement présents dans ces produits.

Diverses solutions ont été proposées pour conditionner de tels produits en évitant toute dégradation des constituants sensibles à l'oxygène, lors de l'opération de stérilisation. Ainsi, dans le document EP 067 420, il est préconisé de réaliser la stérilisation dans un autoclave pressurisé par de l'azote gazeux, et d'embàller le récipient ainsi stérilisé dans un matériau imperméable aux gaz.

Un tel procédé s'avère particulièrement difficile à mettre en oeuvre dans un autoclave industriel et nécessite, pour être efficace, de procéder à l'opération de suremballage dans une atmosphère exempte d'oxygène, de préférence immédiatement après la stérilisation.

De plus, ce procédé ne permet pas de garantir la stérilité de la surface externe du récipient.

Pour assurer la stérilité de la surface externe d'un récipient à base de PVC, lors de son utilisation, il est nécessaire de le "suremballer", préalablement à l'opération de stérilisation, au moyen d'un récipient secondaire clos, formé d'une matière étanche aux micro-organismes.

Cependant, lors de l'opération de stérilisation d'un tel système de conditionnement, la matière plastique à base de PVC constituant le récipient primaire, devient opalescente ce qui empêche toute observation visuelle du contenu du récipient.

Dans le cas où le suremballage ou récipient secondaire est constitué d'une matière perméable aux gaz, ce phénomène est réversible et le retour à un état de transparence est observé après une durée de quelques heures à plusieurs jours. Cependant, une telle matière ne peut être utilisée que dans le seul cas où le produit à conditionner ne contient aucune substance susceptible de se dégrader par contact avec un gaz.

Dans le cas où le produit à conditionner contient au moins une substance susceptible de se dégrader par contact avec un gaz, en particulier l'oxygène de l'air, il est indispensable d'utiliser un suremballage étanche aux gaz pour éviter toute dégradation du produit à conditionner. Mais le phénomène d'opalescence précité est alors irréversible aussi longtemps que le récipient primaire est maintenu à l'intérieur du récipient secondaire formant suremballage.

L'apparence opalescente du récipient primaire à l'ouverture d'un tel système de conditionnement empêche l'inspection visuelle du contenu dudit récipient primaire et s'avère particulièrement gênante, notamment dans le cas de produits pharmaceutiques destinés à être utilisés extemporanément, comme en particulier un produit à administrer par voie intraveineuse.

Le procédé décrit dans le document EP 109 983 permet de résoudre ces difficultés.

Il comprend :
a) le remplissage d'un récipient primaire en matière plastique flexible par un produit liquide à conditionner tel qu'une solution de perfusion ;
b) l'emballage de ce récipient avec un premier matériau constitué d'une matière plastique faiblement perméable au gaz et résistant à la chaleur ;
c) la stérilisation en autoclave de l'ensemble ainsi constitué dans une atmosphère saturée de vapeur et exempte d'oxygène ;
d) le refroidissement de cet ensemble dans des conditions contrôlées de pression par introduction d'un gaz inerte dans l'autoclave ; et
e) l'emballage de l'ensemble ainsi constitué dans un second matériau ayant une forte imperméabilité à l'oxygène gazeux, cette opération étant réalisée sous vide ou dans une atmosphère à base d'azote.

Ce procédé garantit la stérilité en surface externe et la transparence du récipient primaire et assure la stabilité dans le temps du produit à conditionner.

Cependant, ce procédé est relativement complexe, puisque sa mise en oeuvre requiert l'utilisation de trois récipients distincts, à savoir un récipient primaire et deux récipients secondaires d'emballage, et nécessite des modifications importantes de l'autoclave, comme en particulier sa connexion à une source d'alimentation en azote.

Dans ces conditions, la présente invention a pour but de résoudre le problème technique consistant en la fourniture d'un nouveau système de conditionnement stérilisable par autoclave d'un produit aqueux contenant au moins une substance susceptible de se dégrader, du type comprenant un récipient primaire réalisé en une matière plastique à base de PVC, emballé dans un récipient secondaire, qui puisse être réalisé et stérilisé de façon aisée en assurant une totale protection de la substance contre sa dégradation, tant à l'issue de l'opération de stérilisation que dans le temps, et en garantissant la stérilité en surface externe et la transparence du récipient primaire dès l'ouverture du récipient secondaire.

Il a été découvert, de façon tout à fait surprenante et inattendue, qu'il était possible de résoudre ce problème technique d'une façon particulièrement simple, en enveloppant le récipient primaire contenant le produit aqueux dans une matière apte à absorber l'eau, de préférence fibreuse, de préférence encore à base de fibres cellulosiques et, dans le cas d'un produit contenant au moins une substance susceptible de se dégrader par contact avec un gaz, en appauvrissant en ce gaz, l'atmosphère présente entre le récipient primaire et le récipient secondaire dans des conditions garantissant l'absence de contrainte mécanique du récipient secondaire sur le récipient primaire.

Ainsi, selon un premier aspect, la présente invention a pour objet un système de conditionnement stérilisable par autoclave d'un produit aqueux contenant au moins une substance susceptible de se dégrader, du type comprenant un récipient primaire transparent clos contenant ledit produit aqueux et formé d'une matière plastique contenant majoritairement du poly(chlorure de vinyle) et un récipient secondaire clos entourant sans contrainte ledit récipient primaire et formé d'une matière étanche aux microorganismes et apte à protéger ladite substance contre sa dégradation et étanche à la vapeur d'eau, caractérisé en ce qu'il comprend une matière apte à absorber l'eau, de préférence fibreuse, de préférence encore à base de fibres cellulosiques, disposée entre lesdits récipients primaire et secondaire et enveloppant ledit récipient primaire.

Par "matière apte à absorber l'eau", on entend toute matière solide apte à fixer de l'eau, de préférence à l'intérieur d'un volume de ladite matière.

Par "matière sensiblement étanche à la vapeur", on entend toute matière étanche à la vapeur, ou dont la perméabilité à la vapeur est inférieure à la perméabilité à la vapeur de la matière apte à absorber l'eau. Dans le cadre de la présente invention, on utilisera de préférence une matière étanche à la vapeur d'eau.

Le système de conditionnement selon l'invention est particulièrement simple et peut être stérilisé dans un autoclave traditionnel, en garantissant la stérilité de la surface externe du récipient primaire et sa transparence dès l'ouverture du récipient secondaire.

Lorsque le produit aqueux à conditionner contient une substance susceptible de se dégrader par contact avec un gaz, notamment l'oxygène, le récipient secondaire est formé d'une matière imperméable au gaz et l'atmosphère dans l'espace compris entre le récipient primaire et le récipient secondaire est appauvrie en gaz susceptible de provoquer la dégradation de la substance dans des conditions, notamment de pression, garantissant l'absence de contrainte mécanique du récipient secondaire sur le récipient primaire.

Selon un second aspect, la présente invention a pour objet un procédé de fabrication d'un système de conditionnement stérilisable par autoclave d'un produit aqueux contenant au moins une substance susceptible de se dégrader, caractérisé en ce qu'il comprend les étapes consistant à :
- introduire de façon étanche un produit aqueux contenant au moins une substance susceptible de se dégrader dans un récipient primaire transparent clos formé d'une matière plastique contenant majoritairement du poly(chlorure de vinyle) ;
- recouvrir ledit récipient primaire ainsi rempli d'une matière apte à absorber l'eau, de préférence fibreuse, de préférence encore à base de fibres cellulosiques ;
- suremballer l'ensemble ainsi constitué par un récipient secondaire formé d'une matière étanche aux microorganismes et apte à protéger ladite substance contre sa dégradation et sensiblement étanche à la vapeur d'eau ;
- éventuellement dans le cas où le produit aqueux précité contient une substance susceptible de se dégrader par contact avec un gaz, mettre sous vide l'ensemble ainsi constitué et introduire un gaz inerte dans l'espace compris entre le récipient primaire et le récipient secondaire jusqu'à une pression finale minimale suffisante pour éviter, qu'après fermeture du récipient secondaire et retour de l'ensemble du système à la pression atmosphérique, ledit récipient secondaire n'exerce une contrainte mécanique sur le récipient primaire ;
- fermer le récipient secondaire ; et
- éventuellement, soumettre le système ainsi constitué à une opération de stérilisation par autoclave.

La présente invention sera mieux comprise à la lecture de la description explicative qui va suivre.

Le système conforme à la présente invention est particulièrement adapté au conditionnement d'un produit aqueux contenant au moins une substance susceptible de se dégrader par contact avec un gaz, comme par exemple l'oxygène de l'air ou le gaz carbonique, ou sous l'effet d'un rayonnement.

Par "produit aqueux", on entend désigner ici tout produit liquide contenant de l'eau, qui peut se présenter notamment sous la forme d'une solution, d'une émulsion ou d'une suspension, destiné ou non à être stérilisé. les aminophénols, en particulier le paracétamol ; les amines aromatiques ; les aminocétones ; les aminoglucosides ; les phénothiazines ; les dérivés cortisoniques ainsi que les dérivés de la tétracycline comme la doxycycline.

Un tel produit peut être également une solution de perfusion constituée d'un mélange d'acides aminés destiné à la nutrition parentérale ou bien encore un liquide d'enrichissement du sang.

En tant que produit aqueux à conditionner, on peut encore citer les fragments biologiques, les produits de dialyse et de nettoyage des plaies, ainsi que les produits dérivés du sang.

Dans le cadre de la présente invention, le récipient primaire est généralement constitué d'une matière plastique, souple et transparente à base de poly(chlorure de vinyle).

Par "matière plastique à base de poly(chlorure de vinyle)", on entend désigner ici une matière plastique contenant majoritairement du poly(chlorure de vinyle) en association avec des quantités plus au moins importantes d'adjuvants spécifiques tels que stabilisants, lubrifiants, charges variées et plastifiants.

De telles matières sont bien connues de l'homme du métier et largement utilisées, notamment dans le domaine pharmaceutique où elles répondent aux monographies de la Pharmacopée Européenne.

Ces matières présentent une résistance à la chaleur suffisante pour supporter une stérilisation en autoclave et pour être scellées à chaud, par exemple par induction à haute fréquence.

Le récipient secondaire destiné à contenir de façon étanche le récipient primaire est généralement constitué d'une matière apte à protéger une substance contre sa dégradation, notamment par contact avec un gaz ou sous l'effet d'un rayonnement. Cette matière peut être souple ou rigide, transparente ou non.

En tant que matière constituant le récipient secondaire apte à protéger une substance contre sa dégradation par contact avec un gaz ou contre un rayonnement on utilisera une matière présentant une forte imperméabilité aux gaz, notamment à l'oxygène ou au gaz carbonique, comme par exemple les films stratifiés comprenant une couche d'aluminium. Un film particulièrement préféré dans le cadre de l'invention est le produit commercialisé sous la dénomination Quadruplex E 91701 par la Société Pechiney Soplaril Flexible Europe.

De telles matières sont généralement sensiblement étanches à la vapeur d'eau et s'opposent donc à la réversibilité du phénomène d'opalescence de la matière constituant le récipient primaire, qui a été décrit en introduction.

Conformément à la présente invention, le récipient primaire est recouvert d'une matière apte à absorber l'eau. Il s'agira préférentiellement d'une matière fibreuse à base de fibres cellulosiques, comme en particulier un papier, un carton ou un matériau tissé coton.

Des matières se présentant sous la forme de films, telles que des films de polyéthylène, de type MOUSFLEX^{®}, de type sachet aluminium conviennent également dans le cadre de l'invention.

De même, des poudres d'amidon de maïs, de type sucre glace, de cellulose microcristalline (PH 1 025), d'acide citrique ou de déshydratant du type argile peuvent être utilisées en tant que matière apte à absorber l'eau.

De très nombreux types de papiers conviennent dans le cadre de la présente invention et l'on citera notamment les papiers de type journal, kraft, papier de soie, papier à cigarette, papier bible, papier condensateur, papier photographique, papier buvard.

D'une façon actuellement préférée, la matière apte à absorber l'eau sera constituée de papier OPALE 250 gr, et formera une enveloppe entourant le récipient primaire.

Selon un mode de réalisation avantageux de l'invention, cette enveloppe pourra être constituée, dans le domaine pharmaceutique, par la notice servant de support à la communication réglementaire d'une spécialité pharmaceutique.

Selon un mode de réalisation préféré, la matière apte à absorber l'eau sera disposée de telle sorte qu'aucune partie du récipient primaire ne soit en contact direct avec le récipient secondaire.

Dans le cas où le produit à conditionner comporte une substance susceptible de se dégrader par contact avec un gaz, le système de conditionnement selon l'invention est préparé dans des conditions telles que l'atmosphère présente entre le récipient primaire et le récipient secondaire est appauvrie en ce gaz.

Ceci peut être obtenu par exemple en introduisant un gaz inerte entre les récipients primaires et secondaires ou bien encore par mise sous vide de cet espace intercalaire, dans le cas où la matière constituant le récipient secondaire est suffisamment rigide.

Par l'expression "gaz inerte", on entend désigner ici tout gaz ne comportant aucun élément susceptible de provoquer une dégradation du produit à conditionner.

Selon un mode de réalisation actuellement préféré, on parvient à cet objectif en soumettant, préalablement à toute opération de stérilisation par autoclave, l'ensemble constitué du récipient primaire recouvert de la matière apte à absorber l'eau et du récipient secondaire, à une mise sous vide dans des conditions de pression contrôlées suivie de l'introduction d'un gaz inerte, tel que par exemple l'azote dans le cas où le produit à conditionner contient une substance sensible à l'oxydation.

Dans tous les cas, il convient d'opérer dans des conditions garantissant que le récipient secondaire n'exerce aucune contrainte mécanique sur le récipient primaire recouvert afin de permettre à la matière précitée de retenir efficacement l'eau qu'elle aura absorbée.

Un tel système de conditionnement peut être soumis à une stérilisation à l'autoclave (à vapeur ou à ruissellement) dans une installation traditionnelle, sans aucune dégradation de la matière plastique à base de PVC constituant le récipient primaire, et en garantissant la parfaite stérilité de la surface externe de ce dernier.

Ainsi, le récipient primaire sera transparent dès l'ouverture du récipient secondaire, que celle-ci soit réalisée immédiatement, ou plusieurs mois après stérilisation.

Des études de vieillissement accéléré ont permis de constater que le récipient primaire est transparent dès l'ouverture du récipient secondaire six mois après l'opération de stérilisation, le système ayant été maintenu à une température de 40°C.

Ce niveau de performance est donc compatible avec les normes de l'industrie pharmaceutique.

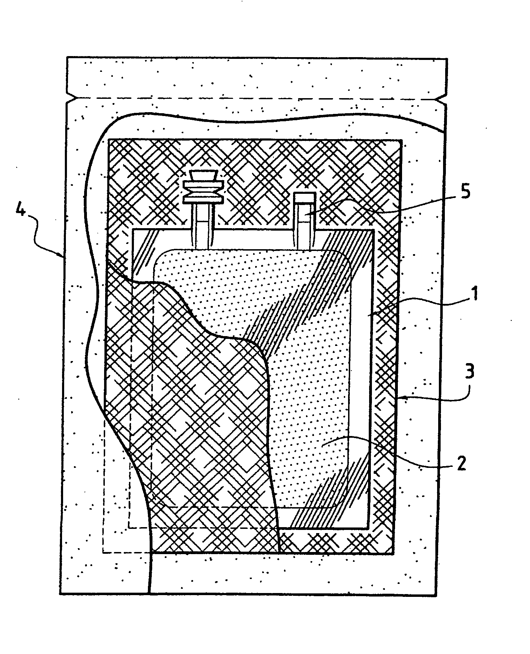

Un procédé de fabrication du système de conditionnement conforme à la présente invention sera maintenant décrit plus en détail, en référence à la figure 1 annexée qui est une vue en plan d'un système de conditionnement conforme à la présente invention.

Pour la clarté de cette description, on utilisera l'exemple d'un produit pharmaceutique tel qu'une solution aqueuse de paracétamol, substance susceptible de se dégrader par oxydation.

Dans ce cas, le récipient primaire 1 est constitué d'une poche en matière plastique souple, transparente à base de PVC comportant un orifice ou une tubulure 5 permettant l'introduction de la solution 2 et susceptible d'être bouché de façon étanche, par exemple à l'aide d'un bouchon en polycarbonate.

Dans l'exemple choisi, cette poche est sensiblement rectangulaire et présente une longueur d'environ 128 mm et une largeur d'environ 80 mm.

Dans une première étape, le récipient primaire est rempli par la solution 2 du produit à conditionner et bouché de façon étanche.

Le récipient primaire ainsi rempli est recouvert par ou enveloppé dans une matière absorbante, constituée par exemple d'une feuille de papier 3, repliée sur elle-même et disposée de telle sorte qu'aucune partie de ce récipient ne puisse être en contact direct ultérieurement avec le récipient secondaire formant suremballage.

Dans l'exemple choisi, cette feuille a une longueur d'environ 190 mm et une largeur d'environ 100 mm.

L'ensemble 1, 3 ainsi constitué est disposé dans un récipient secondaire constitué d'une poche 4 formée d'une matière étanche aux gaz comme par exemple du Quadruplex E 91701.

La poche 4 présente une longueur d'environ 250 mm et une largeur d'environ 140 mm.

Le système de conditionnement ainsi constitué est placé dans une cloche à vide. D'une façon générale, on procède à une mise sous vide jusqu'à une pression maximale permettant d'appauvrir suffisamment en oxygène l'atmosphère dans l'enceinte de la cloche pour assurer la protection contre l'oxydation de la solution aqueuse de paracétamol.

Puis on introduit dans l'enceinte de la cloche un gaz inerte, comme par exemple l'azote, jusqu'à une pression minimale suffisante pour éviter, qu'après fermeture du récipient secondaire et retour de l'ensemble du système à la pression atmosphérique, ledit récipient secondaire, n'exerce une contrainte mécanique sur le récipient primaire.

Dans l'exemple particulier, une pression absolue de l'ordre de 10 mbar a été réalisée avant de remonter la pression dans l'enceinte jusqu'à une valeur supérieure à environ 250 mbars, avec de l'azote. Lorsque la pression en gaz inerte était de l'ordre de 750 mbar, le récipient secondaire a été scellé à chaud.

L'homme du métier déterminera facilement la valeur de pression, lors de la mise sous vide et après introduction du gaz inerte, en fonction de la sensibilité du produit à la dégradation par oxydation et en fonction du volume résiduel existant entre le récipient primaire et le récipient secondaire.

Ce système est alors introduit dans un autoclave à la vapeur traditionnel.

Après stérilisation, l'ensemble est refroidi.

Le récipient primaire est parfaitement transparent et cet état persiste de nombreux mois.

En outre, ce système de conditionnement garantit une totale protection de la substance contre sa dégradation.

Certaines variantes peuvent être apportées au procédé qui vient d'être décrit.

Par exemple, si le récipient secondaire est constitué d'une matière rigide, il n'est pas nécessaire de réintroduire un gaz inerte entre les récipients primaires et secondaires, après l'étape de mise sous vide.

En effet, il a été constaté que l'introduction d'un gaz inerte est seulement nécessaire dans le cas où le récipient secondaire est constitué d'une matière souple, laquelle a tendance à exercer une contrainte forte et à provoquer un contact important entre le récipient primaire et l'enveloppe en papier, au moins dans certaines zones au niveau desquelles la matière plastique à base de PVC a tendance à subir un phénomène d'opalescence.

Le système de conditionnement et son procédé de fabrication qui viennent d'être décrits présentent de nombreux avantages par rapport à l'enseignement de l'état de la technique.

Tout d'abord, ce système peut être stérilisé dans un autoclave standard de stérilisation à la vapeur et ne nécessite aucune modification de ce matériel.

Ensuite, un seul conditionnement secondaire est nécessaire, au lieu de deux comme décrit dans le brevet EP 0 109 983, ce qui conduit à un gain économique tant en ce qui concerne le coût des matières à utiliser qu'en ce qui concerne le coût de mise en oeuvre du procédé de fabrication de conditionnement.

## Revendications

1. Système de conditionnement stérilisable par autoclave d'un produit aqueux contenant au moins une substance susceptible de se dégrader, du type comprenant un récipient primaire (1) transparent clos contenant ledit produit aqueux et formé d'une matière plastique contenant majoritairement du poly(chlorure de vinyle) et un récipient secondaire (4) clos entourant sans contrainte ledit récipient primaire et formé d'une matière étanche aux microorganismes et apte à protéger ladite substance contre sa dégradation et étanche à la vapeur d'eau, **caractérisé en ce qu'**il comprend une matière apte à absorber l'eau (3), de préférence fibreuse, de préférence encore à base de fibres cellulosiques, disposée entre lesdits récipients primaire (1) et secondaire (4) et enveloppant ledit récipient primaire ; ledit conditionnement étant susceptible d'être stérilisé par autoclave sans affecter les propriétés de transparence du récipient primaire (1).

2. Système de conditionnement selon la revendication 1, **caractérisé en ce que** le produit aqueux précité contient une substance susceptible de se dégrader par contact avec un gaz, **en ce que** le récipient secondaire (4) est formé d'une matière imperméable aux gaz et **en ce que** l'atmosphère dans l'espace compris entre le récipient primaire (1) et le récipient secondaire (4) est appauvrie en gaz susceptible de provoquer la dégradation de la substance.

3. Système de conditionnement selon la revendication 1 ou 2, **caractérisé en ce que** la matière apte à absorber l'eau (3) précitée est une matière fibreuse, et en particulier un papier.

4. Système de conditionnement selon l'une des revendications 1 à 3, **caractérisé en ce que** la matière apte à absorber l'eau (3) précitée est disposée de telle sorte qu'aucune partie du récipient primaire (1) ne soit en contact direct avec le récipient secondaire (4).

5. Procédé de fabrication d'un système de conditionnement selon l'une des revendications 1 à 4, **caractérisé en ce qu'**il comprend les opérations consistant à :
- introduire de façon étanche un produit aqueux contenant au moins une substance susceptible de se dégrader dans un récipient primaire (1) transparent clos formé d'une matière plastique contenant majoritairement du poly(chlorure de vinyle) ;
- recouvrir ledit récipient primaire (1) ainsi rempli d'une matière apte à absorber l'eau (3), de préférence fibreuse, de préférence encore à base de fibres cellulosiques ;
- suremballer l'ensemble ainsi constitué par un récipient secondaire (4) formé d'une matière étanche aux microorganismes et apte à protéger ladite substance contre sa dégradation et étanche à la vapeur d'eau ;
- éventuellement mettre sous vide l'ensemble ainsi constitué et introduire un gaz inerte dans l'espace compris entre le récipient primaire et le récipient secondaire (4) jusqu'à une pression finale minimale suffisante pour éviter, qu'après fermeture du récipient secondaire (4) et retour de l'ensemble du système à la pression atmosphérique, ledit récipient secondaire (4) n'exerce une contrainte mécanique sur le récipient primaire (1) ;
- fermer le récipient secondaire (4) ; et
- éventuellement, soumettre le système ainsi constitué à une opération de stérilisation par autoclave.

6. Procédé selon la revendication 5, **caractérisé en ce que** la matière apte à absorber l'eau (3) se présente sous la forme d'enveloppe entourant le récipient primaire (1) et est constituée d'une matière fibreuse, telle qu'en particulier un papier.

7. Procédé selon la revendication 5 ou 6, **caractérisé en ce que** la matière apte à absorber l'eau (3) précitée est disposée de telle sorte qu'aucune partie du récipient primaire (1) ne soit en contact direct avec le récipient secondaire (4).

## Claims

1. Autoclave-sterilizable packaging system for an aqueous product containing at least one substance capable of degrading, of the type comprising a sealed transparent primary container (1) containing said aqueous product and formed from a plastic containing predominantly polyvinyl chloride and a sealed secondary container (4) surrounding, without stress, said primary container and formed from a material that is impermeable to microorganisms and capable of protecting said substance against its degradation and that is impermeable to steam, **characterized in that** it comprises a material capable of absorbing water (3), preferably that is fibrous, more preferably that is based on cellulose fibres, which is positioned between said primary container (1) and secondary container (4) and which surrounds said primary container; said packaging being capable of being sterilized by autoclave without adversely affecting the transparency properties of the primary container (1).

2. Packaging system according to Claim 1, **characterized in that** the abovementioned aqueous product contains a substance capable of degrading by contact with a gas, **in that** the secondary container (4) is formed from a material that is impermeable to gases and **in that** the atmosphere in the space between the primary container (1) and the secondary container (4) is depleted of gas capable of causing the degradation of the substance.

3. Packaging system according to Claim 1 or 2, **characterized in that** the abovementioned material capable of absorbing water (3) is a fibrous material, and in particular a paper.

4. Packaging system according to one of Claims 1 to 3, **characterized in that** the abovementioned material capable of absorbing water (3) is positioned such that no part of the primary container (1) is in direct contact with the secondary container (4).

5. Process for manufacturing a packaging system according to one of Claims 1 to 4, **characterized in that** it comprises the operations consisting in:
- introducing, in a leaktight manner, an aqueous product containing at least one substance capable of degrading into a sealed transparent primary container (1) formed from a plastic containing predominantly polyvinyl chloride;
- covering said thus filled primary container (1) with a material capable of absorbing water (3), which is preferably fibrous, more preferably based on cellulose fibres;
- overwrapping the assembly thus formed with a secondary container (4) formed from a material that is impermeable to microorganisms and capable of protecting said substance against its degradation and that is impermeable to steam;
- optionally putting the assembly thus formed under vacuum and introducing an inert gas into the space between the primary container and the secondary container (4) up to a minimum final pressure sufficient to prevent, after closure of the secondary container (4) and returning the whole of the system to atmospheric pressure, said secondary container (4) from exerting a mechanical stress on the primary container (1) ;
- closing the secondary container (4); and
- optionally, subjecting the system thus formed to an autoclave sterilization operation.

6. Process according to Claim 5, **characterized in that** the material capable of absorbing water (3) is present in the form of an envelope surrounding the primary container (1) and is constituted of a fibrous material, such as in particular a paper.

7. Process according to Claim 5 or 6, **characterized in that** the abovementioned material capable of absorbing water (3) is positioned so that no part of the primary container (1) is in direct contact with the secondary container (4).

## Patentansprüche

1. Durch Autoklaven sterilisierbares Verpackungssystem für ein wäßriges Produkt, das wenigstens eine verderbliche Substanz enthält, vom Typ umfassend einen transparenten, geschlossenen Primärbehälter (1), der das wäßrige Produkt enthält und von einem Kunststoff gebildet ist, der mehrheitlich Polyvinylchlorid enthält, sowie einen geschlossenen Sekundärbehälter (4), der den Primärbehälter spannungsfrei umschließt und von einem Material gebildet ist, welches gegenüber Mikroorganismen dicht und geeignet ist, die Substanz vor ihrem Verderb zu schützen, und wasserdampfdicht ist, **dadurch gekennzeichnet, daß** es ein wasserabsorptionsfähiges Material (3), vorzugsweise Fasermaterial, weiterhin vorzugsweise auf der Basis von Zellulosefasern umfaßt, das zwischen dem Primärbehälter (1) und dem Sekundärbehälter (4) angeordnet ist und den Primärbehälter umschließt, wobei die Verpackung geeignet ist, durch Autoklaven sterilisiert zu werden, ohne die Transparenzeigenschaften des Primärbehälters (1) zu beeinträchtigen.

2. Verpackungssystem nach Anspruch 1, **dadurch gekennzeichnet, daß** das vorgenannte wäßrige Produkt eine durch Kontakt mit einem Gas verderbliche Substanz enthält, daß der Sekundärbehälter (4) von einem gasundurchlässigen Material gebildet ist und daß die Atmosphäre in dem Raum zwischen dem Primärbehälter (1) und dem Sekundärbehälter (4) an Gas, das geeignet ist, das Verderben der Substanz zu bewirken, verarmt ist.

3. Verpackungssystem nach Anspruch 1 oder 2, **dadurch gekennzeichnet, daß** das vorgenannte wasserabsorptionsfähige Material (3) ein Fasermaterial und insbesondere ein Papier ist.

4. Verpackungssystem nach einem der Ansprüche 1 bis 3, **dadurch gekennzeichnet, daß** das vorgenannte wasserabsorptionsfähige Material (3) derart angeordnet ist, daß kein Teil des Primärbehälters (1) in direktem Kontakt mit dem Sekundärbehälter (4) steht.

5. Verfahren zur Herstellung eines Verpackungssystems nach einem der Ansprüche 1 bis 4, **dadurch gekennzeichnet, daß** es die Arbeitsschritte umfaßt, die darin bestehen:
- ein wäßriges Produkt, das wenigstens eine verderbliche Substanz enthält, auf dichte Weise in einen transparenten, geschlossenen Primärbehälter (1) einzuleiten, der von einem mehrheitlich Polyvinylchlorid enthaltenden Kunststoff gebildet ist,
- den so gefüllten Primärbehälter (1) mit einem wasserabsorptionsfähigen Material (3), vorzugweise Fasermaterial, weiterhin vorzugsweise auf der Basis von Zellulosefasern zu bedecken,
- die so gebildete Anordnung mit einem Sekundärbehälter (4), der von einem Material gebildet ist, welches gegenüber Mikroorganismen dicht und geeignet ist, die Substanz vor ihrem Verderb zu schützen, und wasserdampfdicht ist, umzuverpacken,
- die so gebildete Anordnung eventuell zu evakuieren und in den Raum zwischen dem Primärbehälter und dem Sekundärbehälter (4) ein Inertgas bis zu einem Mindestenddruck einzuleiten, der ausreichend ist, um zu vermeiden, daß nach Verschließen des Sekundärbehälters (4) und Rückkehr der Anordnung des Systems zum atmosphärischen Druck, der Sekundärbehälter (4) auf den Primärbehälter (1) eine mechanische Spannung ausübt,
- den Sekundärbehälter (4) zu verschließen und
- das so gebildete System eventuell einem Schritt zur Sterilisation durch Autoklaven zu unterziehen.

6. Verfahren nach Anspruch 5, **dadurch gekennzeichnet, daß** das wasserabsorptionsfähige Material (3) in Form einer den Primärbehälter (1) umschließenden Hülle vorliegt und von einem Fasermaterial, wie insbesondere einem Papier gebildet ist.

7. Verfahren nach Anspruch 5 oder 6, **dadurch gekennzeichnet, daß** das vorgenannte wasserabsorptionsfähige Material (3) derart angeordnet ist, daß kein Teil des Primärbehälters (1) in direktem Kontakt mit dem Sekundärbehälter (4) steht.
